# EUROPEAN PATENT SPECIFICATION

(11) **EP 1 633 730 B1**
(45) Date of publication and mention of the grant of the patent: **20.01.2010**
(21) Application number: 04735275.2
(22) Date of filing: 28.05.2004
(51) Int. Cl.: C07D 277/62, C07D 417/12, C07D 263/56, C07D 413/12, C07D 277/82, C07D 277/68, A01N 43/76, A01N 43/78

(54) **N-ALKYNYL-2-HETEROARYLOXYALKYLAMIDES FOR USE AS FUNGICIDES**
N-ALKYNYL-2-HETEROARYLOXYALKYLAMIDE ZUR VERWENDUNG ALS FUNGIZIDE
N-ALKYNYL-2-HETEROARYLOXYALKYLAMIDES UTILISES COMME FONGICIDES

(30) Priority: 04.06.2003 GB 0312864
(43) Date of publication of application: 15.03.2006
(73) Proprietor: Syngenta Limited, Guildford Surrey GU2 7YH (GB)
(72) Inventor: SALMON, Roger, c/o Syngenta Limited, Bracknell, Berks RG42 6EY (GB); CROWLEY, Patrick, Jelf, c/o Syngenta Limited, Bracknell, Berks RG42 6EY (GB)
(74) Representative: Ward, Steven Paul
(86) International application number: PCT/GB2004/002308
(87) International publication number: WO 2004/108694

(56) References cited:
- US-A- 4 784 682
- PATENT ABSTRACTS OF JAPAN vol. 2000, no. 21, 3 August 2001 (2001-08-03) -& JP 2001 089453 A (KUMIAI CHEM IND CO LTD; IHARA CHEM IND CO LTD), 3 April 2001 (2001-04-03)

## Description

This invention relates to novel *N-*alkynyl-2-heteroaryloxyalkylamides, to processes for preparing them, to compositions containing them and to methods of using them to combat fungi, especially fungal infections of plants.

Various quinolin-8-oxyalkanecarboxylic acid derivatives are described as being useful as antidotes for herbicides or as herbicide safeners (see, for example, US 4,881,966, US 4,902,340 and US 5,380,852). Certain pyridyl- and pyrimidinyloxy-(thio)alkanoic acid amide derivatives are described in, for example, WO 99/33810 and US 6090815, together with their use as agricultural and horticultural fungicides. In addition, certain phenoxyalkanoic acid amide derivatives are described in, for example, US 4116677 and US 4168319, together with their use as herbicides and mildewicides.

Certain 2-benzothiazolyl- and 2-benzoxazolyloxyalkylamides wherein the amide group may contain unsubstituted ethynyl are disclosed in JP 2001 A08 9453 and further 2- benzoxazolyloxyalkylamides with phenyl substituents in the amide group are known from US 4 784 682.

According to the present invention, there is provided a compound of the general formula (1): 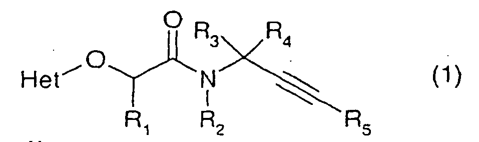 wherein Het is 5 or 6 benzothiazolyl optionally bearing a 2-C substituent, 5-(2,1-benzisothiazolyl) optionally bearing a 3-C substituent, 6-benzoxazolyl optionally bearing a 2-C substituent. 5-(2,1-benzisoxazolyl) optionally bearing a 3-C substituent, 6-(1*H-*benzimidazolyl) optionally bearing a 2-C substituent and optibnally bearing a *N*-C1-4 alkyl substituent, 5-(1*H*-indazolyl) optionally bearing a 3-C substitutent and optionally bearing a *N*-C₁₋₄ alkyl substituent, 6-(1,2,3-benzothiadiazolyl) or 6-(1,2,3-benzoxa-diazolyl), wherein any of the foregoing optional substituents are selected from halo, C₁₋₄ alkyl, C₁₋₄ alkoxy, C₁₋₄ alkylthio, C₁₋₄ alkylsulphinyl, C₁₋₄ alkylsulphonyl, halo(C₁₋₄)alkyl or balo(C₁₋₄)alkoxy, halo(C₁₋₄)alkylthio, halo(C₁₋₄)alkylsulphinyl, halo(C₁₋₄)alkylsulphonyl or mono- or di-(C₁₋₄) alkylamino; R₁ is methyl, ethyl, *n*-propyl, 2,2,2-trifluoro-methyl, cyanomethyl, acetylmethyl, methoxycarbonylmethyl, methoxycarbonylethyl, hydroxymethyl, hydroxyethyl, methoxymethyl, methylthiomethyl, ethoxymethyl, 2-methoxyethyl, methoxy, ethoxy, *n-*propoxy or *n-*butoxy R₂ is H; R₃ and R₄ are both methyl; and R₅ is methyl, hydroxymethyl, methoxymethyl, 1-methoxyethyl, 3-cyano-*n*-propyl or *tert*-butyldimethylsiloxymethyl.

The compounds of the invention contain at least one asymmetric carbon atom (and at least two when R₃ and R₄ are different) and may exist as enantiomers (or as pairs of diastereoisomers) or as mixtures of such. However, these mixtures may be separated into individual isomers or isomer pairs, and this invention embraces such isomers and mixtures thereof in all proportion. It is to be expected that for any given compound, one isomer may be more fungicidally active than another. Preferably R₁ is ethyl, methoxy, ethoxy or methoxymethyl, especially ethyl. Preferably R₅ is methyl or methoxymethyl.

In yet another aspect, the invention provides a compound of the general formula (1) wherein Het is 5- or 6-benzothiazolyl, 5-(2,1-benzisothiazolyl), 6-benzoxazolyl, 5-(2,1-benzisoxazolyl), 6-(1*H*-benzimidazolyl) optionally bearing a *N*-C₁₋₄ alkyl substituents, 5-(1*H*-indazolyl) optionally bearing a *N*-C₁₋₄ alkyl substituent, 6-(1,2,3-benzothiadiazolyl) or 6-(1,2,3-benzoxadiazolyl); R₁ is methyl, ethyl, *n*-propyl, 2,2,2-trifluoromethyl, cyanomethyl, acetylmethyl, methoxycarbonylmethyl, methoxycarbonylethyl, hydroxymethyl, hydroxyethyl, methoxymethyl, methylthiomethyl, ethoxymethyl, 2-methoxyethyl, methoxy, ethoxy, *n*-propoxy or *n*-butoxy; R₂ is H; R₃ and R₄ are both methyl; and R₅ is methyl, hydroxymethyl, methoxymethyl, 1-methoxyethyl, 3-cyano-*n*-propyl or *tert*-butyldimethylsiloxymethyl. Preferably R₁ is ethyl, methoxy, ethoxy or methoxymethyl, especially ethyl. Preferably R₅ is methyl or methoxymethyl.

Compounds that form part of the invention are illustrated in Tables 1 to 62 below.

The compounds in Table 1 are of the general formula (1) where Het is 6-benzothiazolyl, R₁ is ethyl, R₂ is H, R₃ and R₄ are both methyl and R₅ has the values given in the table.

**Table 1**

| **Compound No.** | **R₅** |
|---|---|
| 2 | CH₃ |
| 10 | HOCH₂ |
| 12 | CH₃OCH₂ |
| 13 | CH₃OCH₂CH₂ |
| 21 | NC-*n*-C₃H₆ |
| 50 | *tert*-C₄H₉(CH₃)₂SiOCH₂ |

### Table 2

Table 2 consists of 92 compounds of the general formula (1), where Het is 6-benzothiazolyl, R₁ is methyl, R₂ is hydrogen, R₃ and R₄ are both methyl and R₅ has the values listed in Table 1. Thus compound 2 of Table 2 is the same as compound 2 of Table 1 except that in compound 2 of Table 2 R₁ is methyl instead of ethyl. Similarly, compounds 10, 12, 13, 21 and 50 of Table 2 are the same as compounds 10, 12, 13, 21 and 50 of Table 1, respectively, except that in the compounds of Table 2 R₁ is methyl instead of ethyl.

### Table 3

Table 3 consists of 92 compounds of the general formula (1), where Het is 6-benzothiazolyl, R₁ is *n*-propyl, R₂ is hydrogen, R₃ and R₄ are both methyl and R₅ has the values listed in Table 1.

### Table 5

Table 5 consists of 92 compounds of the general formula (1), where Het is 6-benzothiazolyl, R₁ is cyanomethyl, R₂ is hydrogen, R₃ and R₄ are both methyl and R₅ has the values listed in Table 1.

### Table 6

Table 6 consists of 92 compounds of the general formula (1), where Het is 6-benzothiazolyl, R₁ is acetylmethyl, R₂ is hydrogen, R₃ and R₄ are both methyl and R₅ has the values listed in Table 1.

### Table 7

Table 7 consists of 92 compounds of the general formula (1), where Het is 6-benzothiazolyl, R₁ is methoxycarbonylmethyl, R₂ is hydrogen, R₃ and R₄ are both methyl and R₅ has the values listed in Table 1.

### Table 8

Table 8 consists of 92 compounds of the general formula (1), where Het is 6-benzothiazolyl, R₁ is methoxycarbonylethyl, R₂ is hydrogen, R₃ and R₄ are both methyl and R₅ has the values listed in Table 1.

### Table 9

Table 9 consists of 92 compounds of the general formula (1), where Het is 6-benzothiazolyl, R₁ is hydroxymethyl, R₂ is hydrogen, R₃ and R₄ are both methyl and R₅ has the values listed in Table 1.

### Table 10

Table 10 consists of 92 compounds of the general formula (1), where Het is 6-benzothiazolyl, R₁ is hydroxethyl, R₂ is hydrogen, R₃ and R₄ are both methyl and R₅ has the values listed in Table 1.

### Table 11

Table 11 consists of 92 compounds of the general formula (1), where Het is 6-benzothiazolyl, R₁ is methoxymethyl, R₂ is hydrogen, R₃ and R₄ are both methyl and R₅ has the values listed in Table 1.

### Table 12

Table 12 consists of 92 compounds of the general formula (1), where Het is 6-benzothiazolyl, R₁ is methylthiomethyl, R₂ is hydrogen, R₃ and R₄ are both methyl and R₅ has the values listed in Table 1.

### Table 13

Table 13 consists of 92 compounds of the general formula (1), where Het is 6-benzothiazolyl, R₁ is ethoxymethyl, R₂ is hydrogen, R₃ and R₄ are both methyl and R₅ has the values listed in Table 1.

### Table 14

Table 14 consists of 92 compounds of the general formula (1), where Het is 6-benzothiazolyl, R₁ is 2-methoxyethyl, R₂ is hydrogen, R₃ and R₄ are both methyl and R₅ has the values listed in Table 1.

### Table 16

Table 16 consists of 92 compounds of the general formula (1), where Het is 6-benzothiazolyl, R₁ is methoxy, R₂ is hydrogen, R₃ and R₄ are both methyl and R₅ has the values listed in Table 1.

### Table 17

Table 17 consists of 92 compounds of the general formula (1), where Het is 6-benzothiazolyl, R₁ is ethoxy, R₂ is hydrogen, R₃ and R₄ are both methyl and R₅ has the values listed in Table 1.

### Table 18

Table 18 consists of 92 compounds of the general formula (1), where Het is 6-benzothiazolyl, R₁ is *n-*propoxy, R₂ is hydrogen, R₃ and R₄ are both methyl and R₅ has the values listed in Table 1.

### Table 19

Table 19 consists of 92 compounds of the general formula (1), where Het is 6-benzothiazolyl, R₁ is *n*-butoxy, R₂ is hydrogen, R₃ and R₄ are both methyl and R₅ has the values listed in Table 1.

### Table 20

Table 20 consists of 92 compounds of the general formula (1), where Het is 5-benzothiazolyl, R₁ is ethyl, R₂ is hydrogen, R₃ and R₄ are both methyl and R₅ has the values listed in Table 1.

### Table 21

Table 21 consists of 92 compounds of the general formula (1), where Het is 5-benzothiazolyl, R₁ is methyl, R₂ is hydrogen, R₃ and R₄ are both methyl and R₅ has the values listed in Table 1.

### Table 22

Table 22 consists of 92 compounds of the general formula (1), where Het is 5-benzothiazolyl, R₁ is *n*-propyl, R₂ is hydrogen, R₃ and R₄ are both methyl and R₅ has the values listed in Table 1.

### Table 24

Table 24 consists of 92 compounds of the general formula (1), where Het is 5-benzothiazolyl, R₁is cyanomethyl, R₂ is hydrogen, R₃ and R₄ are both methyl and R₅ has the values listed in Table 1.

### Table 25

Table 25 consists of 92 compounds of the general formula (1), where Het is 5-benzothiazolyl, R₁ is acetylmethyl, R₂ is hydrogen, R₃ and R₄ are both methyl and R₅ has the values listed in Table 1.

### Table 26

Table 26 consists of 92 compounds of the general formula (1), where Het is 5-benzothiazolyl, R₁ is methoxycarbonylmethyl, R₂ is hydrogen, R₃ and R₄ are both methyl and R₅ has the values listed in Table 1.

### Table 27

Table 27 consists of 92 compounds of the general formula (1), where Het is 5-benzothiazolyl, R₁ is methoxycarbonylethyl, R₂ is hydrogen, R₃ and R₄ are both methyl and R₅ has the values listed in Table 1.

### Table 28

Table 28 consists of 92 compounds of the general formula (1), where Het is 5-benzothiazolyl, R₁ is hydroxymethyl, R₂ is hydrogen, R₃ and R₄ are both methyl and R₅ has the values listed in Table 1.

### Table 29

Table 29 consists of 92 compounds of the general formula (1), where Het is 5-benzothiazolyl, R₁ is hydroxethyl, R₂ is hydrogen, R₃ and R₄ are both methyl and R₅ has the values listed in Table 1.

### Stable 30

Table 30 consists of 92 compounds of the general formula (1), where Het is 5-benzothiazolyl, R₁ is methoxymethyl, R₂ is hydrogen, R₃ and R₄ are both methyl and R₅ has the values listed in Table 1.

### Table 31

Table 31 consists of 92 compounds of the general formula (1), where Het is 5-benzothiazolyl, R₁ is methylthiomethyl, R₂ is hydrogen, R₃ and R₄ are both methyl and R₅ has the values listed in Table 1.

### Table 32

Table 32 consists of 92 compounds of the general formula (1), where Het is 5-benzothiazolyl, R₁ is ethoxymethyl, R₂ is hydrogen, R₃ and R₄ are both methyl and R₅ has the values listed in Table 1.

### Table 33

Table 33 consists of 92 compounds of the general formula (1), where Het is 5-benzothiazolyl, R₁ is 2-methoxyethyl, R₂ is hydrogen, R₃ and R₄ are both methyl and R₅ has the values listed in Table 1.

### Table 35

Table 35 consists of 92 compounds of the general formula (1), where Het is 5-benzothiazolyl, R₁ is methoxy, R₂ is hydrogen, R₃ and R₄ are both methyl and R₅ has the values listed in Table 1.

### Table 36

Table 36 consists of 92 compounds of the general formula (1), where Het is 5-benzothiazolyl, R₁ is ethoxy, R₂ is hydrogen, R₃ and R₄ are both methyl and R₅ has the values listed in Table 1.

### Table 37

Table 37 consists of 92 compounds of the general formula (1), where Het is 5-benzothiazolyl, R₁ is *n*-propoxy, R₂ is hydrogen, R₃ and R₄ are both methyl and R₅ has the values listed in Table 1.

### Table 38

Table 38 consists of 92 compounds of the general formula (1), where Het is 5-benzothiazolyl, R₁ is *n*-butoxy, R₂ is hydrogen, R₃ and R₄ are both methyl and R₅ has the values listed in Table 1.

### Table 39

Table 39 consists of 92 compounds of the general formula (1), where Het is 6-benzoxazolyl, R₁ is ethyl, R₂ is hydrogen, R₃ and R₄ are both methyl and R₅ has the values listed in Table 1.

### Table 40

Table 40 consists of 92 compounds of the general formula (1), where Het is 6-benzoxazolyl, R₁ is methyl, R₂ is hydrogen, R₃ and R₄ are both methyl and R₅ has the values listed in Table 1.

### Table 41

Table 41 consists of 92 compounds of the general formula (1), where Het is 6-benzoxazolyl, R₁ is *n*-propyl, R₂ is hydrogen, R₃ and R₄ are both methyl and R₅ has the values listed in Table 1.

### Table 43

Table 43 consists of 92 compounds of the general formula (1), where Het is 6-benzoxazolyl, R₁ is cyanomethyl, R₂ is hydrogen, R₃ and R₄ are both methyl and R₅ has the values listed in Table 1.

### Table 44

Table 44 consists of 92 compounds of the general formula (1), where Het is 6-benzoxazolyl, R₁ is acetylmethyl, R₂ is hydrogen, R₃ and R₄ are both methyl and R₅ has the values listed in Table 1.

### Table 45

Table 45 consists of 92 compounds of the general formula (1), where Het is 6-benzoxazolyl, R₁ is methoxycarbonylmethyl, R₂ is hydrogen, R₃ and R₄ are both methyl and R₅ has the values listed in Table 1.

### Table 46

### Table 46 consists of 92 compounds of the general formula (1), where Het is 6-benzoxa-zolyl, R₁ is methoxycarbonylethyl, R₂ is hydrogen, R₃ and R₄ are both methyl and R₅ has the values listed in Table 1.

### Table 47

Table 47 consists of 92 compounds of the general formula (1), where Het is 6-benzoxazolyl, R₁ is hydroxymethyl, R₂ is hydrogen, R₃ and R₄ are both methyl and R₅ has the values listed in Table 1.

### Table 48

Table 48 consists of 92 compounds of the general formula (1), where X is Het is 6-benzoxazolyl, R₁ is hydroxethyl, R₂ is hydrogen, R₃ and R₄ are both methyl and R₅ has the values listed in Table 1.

### Table 49

Table 49 consists of 92 compounds of the general formula (1), where Het is 6-benzoxazolyl, R₁ is methoxymethyl, R₂ is hydrogen, R₃ and R₄ are both methyl and R₅ has the values listed in Table 1.

### Table 50

Table 50 consists of 92 compounds of the general formula (1), where Het is 6-benzoxazolyl, R₁ is methylthiomethyl, R₂ is hydrogen, R₃ and R₄ are both methyl and R₅ has the values listed in Table 1.

### Table 51

Table 51 consists of 92 compounds of the general formula (1), where Het is 6-benzoxazolyl, R₁ is ethoxymethyl, R₂ is hydrogen, R₃ and R₄ are both methyl and R₅ has the values listed in Table 1.

### Table 52

Table 52 consists of 92 compounds of the general formula (1), where Het is 6-benzoxazolyl, R₁ is 2-methoxyethyl, R₂ is hydrogen, R₃ and R₄ are both methyl and R₅ has the values listed in Table 1.

### Table 54

Table 54 consists of 92 compounds of the general formula (1), where Het is 6-benzoxazolyl, R₁ is methoxy, R₂ is hydrogen, R₃ and R₄ are both methyl and R₅ has the values listed in Table 1.

### Table 55

Table 55 consists of 92 compounds of the general formula (1), where Het is 6-benzoxazolyl, R₁ is ethoxy, R₂ is hydrogen, R₃ and R₄ are both methyl and R₅ has the values listed in Table 1.

### Table 56

Table 56 consists of 92 compounds of the general formula (1), where Het is 6-benzoxazolyl, R₁ is *n*-propoxy, R₂ is hydrogen, R₃ and R₄ are both methyl and R₅ has the values listed in Table 1.

### Table 57

Table 57 consists of 92 compounds of the general formula (1), where Het is 6-benzoxazolyl, R₁ is *n*-butoxy, R₂ is hydrogen, R₃ and R₄ are both methyl and R₅ has the values listed in Table 1.

### Table 58

Table 58 consists of 92 compounds of the general formula (1), where Het is 2-methyl-6-benzothiazolyl, R₁ is ethyl, R₂ is hydrogen, R₃ and R₄ are both methyl and R₅ has the values listed in Table 1.

### Table 59

Table 59 consists of 92 compounds of the general formula (1), where Het is 2-methyl-amino-6-benzothiazolyl, R₁ is ethyl, R₂ is hydrogen, R₃ and R₄ are both methyl and R₅ has the values listed in Table 1.

### Table 60

Table 60 consists of 92 compounds of the general formula (1), where Het is 2-chloro-6-benzothiazolyl, R₁ is ethyl, R₂ is hydrogen, R₃ and R₄ are both methyl and R₅ has the values listed in Table 1.

### Table 61

Table 61 consists of 92 compounds of the general formula (1), where Het is 2-methyl-6-benzoxazolyl, R₁ is ethyl, R₂ is hydrogen, R₃ and R₄ are both methyl and R₅ has the values listed in Table 1.

### Table 62

Table 62 consists of 92 compounds of the general formula (1), where Het is 2-methyl-5-benzothiazolyl, R₁ is ethyl, R₂ is hydrogen, R₃ and R₄ are both methyl and R₅ has the values listed in Table 1.

The compounds of formula (1) may be prepared as outlined in Schemes 1 to 9 below in which Het, W, X, Y, Z, R₁, R₂, R₃, R₄ and R₅ have the meanings given above, R₆ is straight-chain C₁₋₄ alkyl, R₇, R₈ and R₉ are independently H or C₁₋₄ alkyl, L is a leaving group such as a halide, for example iodide, an alkyl- or arylsulphonyloxy group, for example methylsulphonyloxy and tosyloxy or a triflate, Hal is halogen, Rₐ is hydrogen or C₁₋₃ alkyl, R_{b} is hydrogen or C₁₋₃ alkyl, provided that the total number of carbon atoms in Rₐ and R_{b} do not exceed three, R_{c} is C₁₋₆ alkyl, optionally substituted benzyl or optionally substituted thienylmethyl and R_{d} has the meaning ascribed to it in the text.

As shown in Scheme 1, the compounds of general formula (1) may be prepared by reacting a compound of the general formula (2), in which the OH group is in the 5- or 6-position of the Het ring system, with a compound of the general formula (3) in the presence of a base in a suitable solvent. Typical solvents include *N,N*-dimethylformamide, *tert*-butanol and *N-*methylpyrrolidin-2-one. Suitable bases include potassium carbonate, potassium *tert-*butoxide, sodium hydride or di*iso*propylethylamine.

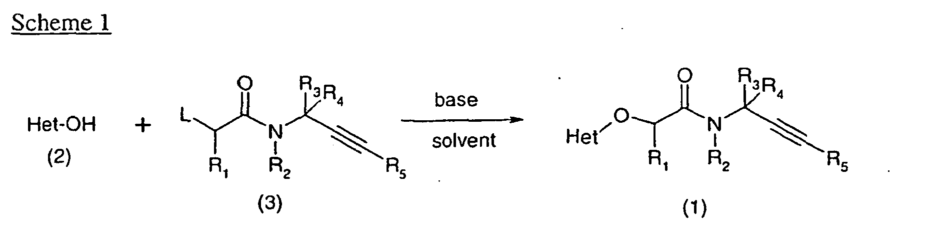

As shown in Scheme 2, compounds of the general formula (3) may be prepared by reacting an amine of the general formula (5) with an acid halide of the general formula (4), or the corresponding acid anhydride, in the presence of a suitable inorganic or organic base, such as potassium carbonate or diisopropylethylamine, in a solvent such as dichloromethane or tetrahydrofuran.

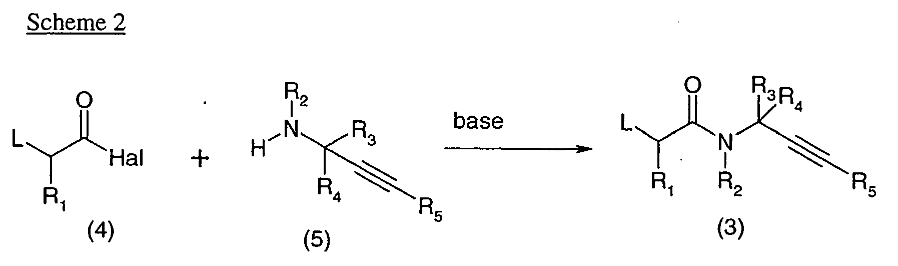

As shown in Scheme 3, amines of the general formula (5), wherein R₂ is H, correspond to amines of the general formula (9) and may be prepared by alkylation of a silyl-protected aminoalkyne of the general formula (7) using a suitable base, such as *n-*butyl lithium, followed by reaction with a suitable alkylating reagent R₅L, such as an alkyl iodide, for example, methyl iodide or 3-chloro-1-iodo-propane, to form an alkylated compound of the general formula (8). In a similar procedure, a silyl-protected aminoalkyne of the general formula (7) may be reacted with a carbonyl derivative RₐCOR_{b}, for example formaldehyde, using a suitable base, such as *n-*butyl lithium, to provide an aminoalkyne (8) containing a hydroxyalkyl moiety. The silyl protecting group may then be removed from a compound of the general formula (8) with, for example, an aqueous acid to form an aminoalkyne of the general formula (9). Aminoalkynes of the general formula (9) may be further derivatised, for instance when R₅ is a hydroxyalkyl group, for example, by reacting a compound of the general formula (9) with a silylating agent, for example t-butyldimethylsilyl chloride, to give a derivative silylated on oxygen of the general formula (9a). In addition, a compound of the general formula (9) may be treated with a base, such as sodium hydride or potassium *bis*(trimethylsilyl)amide followed by a compound R_{c}L to give a compound of the general formula (9b). In an alternative sequence, a compound of general formula (8) may be treated with a base, such as sodium or potassium *bis*(trimethylsilyl)amide, followed by a compound R_{c}L, where L represents a halogen or sulphonate ester such as OSO₂Me, or OSO₂-4-tolyl, for example ethyl iodide, to give, after removal of the silyl protecting group, compounds of general formula (9b).

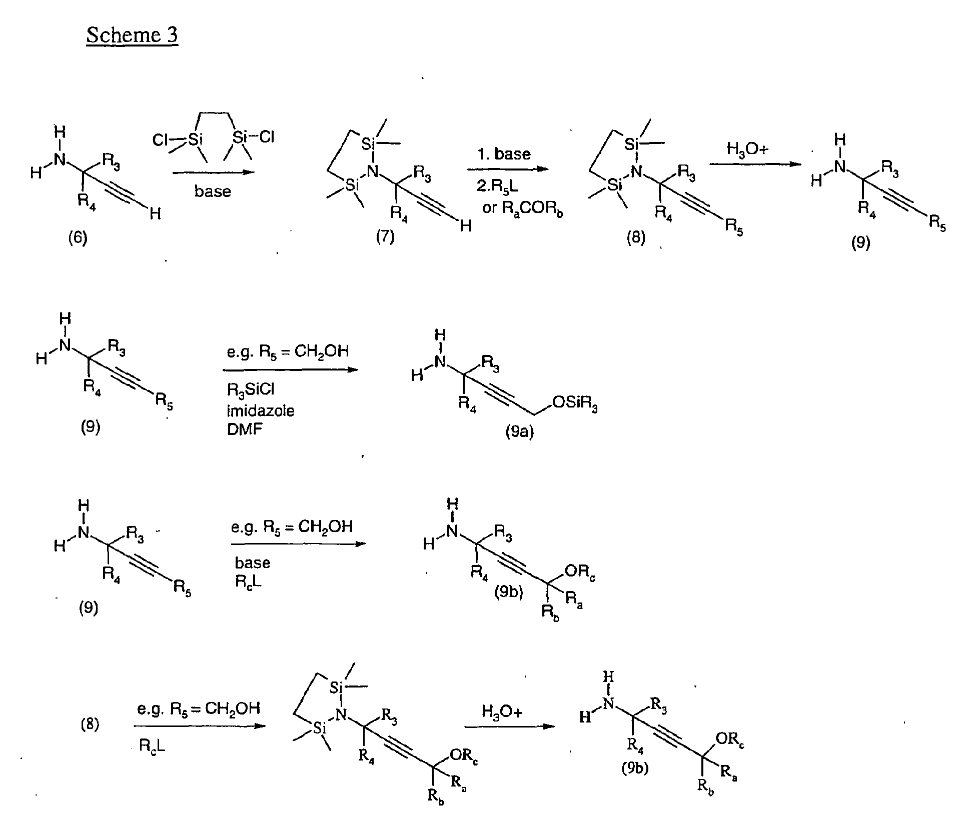

Silyl-protected aminoalkynes of the general formula (7) may be obtained by reacting amines of general formula (6) with 1,2-*bis*-(chlorodimethylsilyl)ethane in the presence of a suitable base, such as a tertiary organic amine base, for example, triethylamine.

Amines of the general formula (6) are either commercially available or may be prepared by standard literature methods (see, for example, EP-A-0834498).

Alternatively, as shown in Scheme 4, compounds of the general formula (1) may be prepared by condensing a compound of the general formula (11), wherein R_{d} is H with an amine of the general formula (5) using suitable activating reagents such as 1-hydroxybenzotriazole and *N*-(3-dimethylaminopropyl)-*N'*-ethyl-carbodiimide hydrochloride.

Where R₂ is other than hydrogen, the R₂ group may be introduced into an aminoalkyne of the general formula (9) by known techniques to form an amine of the general formula (5).

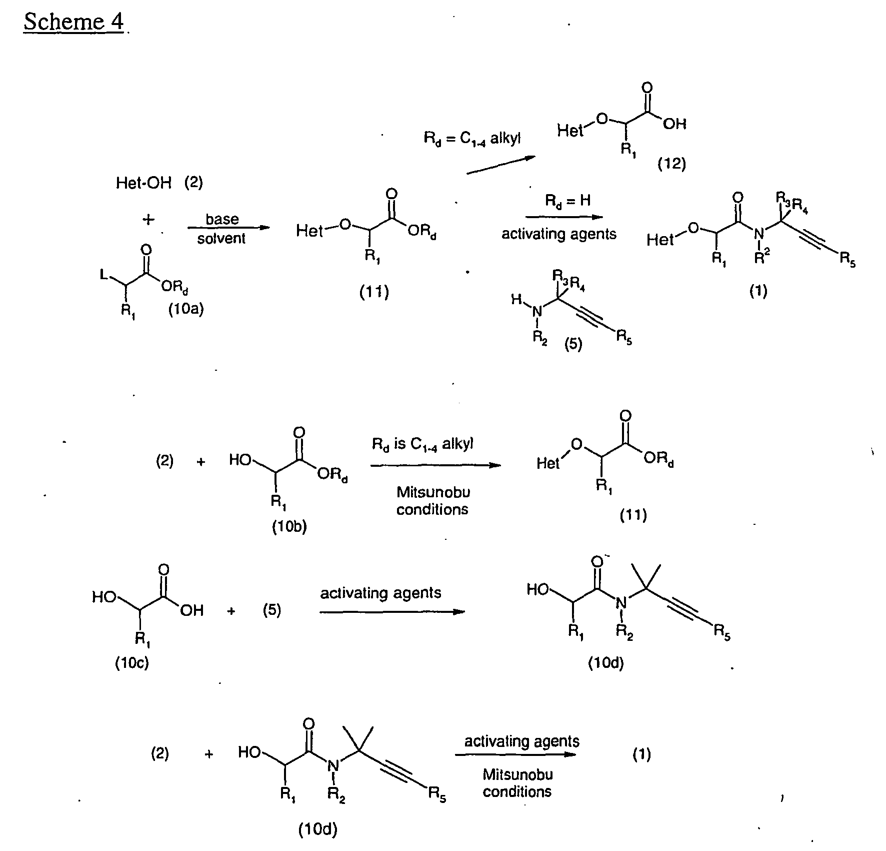

Compounds of the general formula (12) may be prepared by the hydrolysis of the corresponding esters of general formula (11), wherein R_{d} is C₁₋₄ alkyl, using known techniques. The esters of the general formula (11), wherein R_{d} is C₁₋₄ alkyl and also acids of the general formula (11), wherein R_{d} is H, may be prepared by reacting a compound of the general formula (2) with an ester or acid of the general formula (10a) in the presence of a suitable base, such as potassium carbonate or sodium hydride, in a suitable solvent, such as *N,N*-dimethylformamide. The esters or acids of the general formula (10a) are either commercially available or may be prepared by standard literature methods from commercially available materials.

Alternatively, as shown in Scheme 4, compounds of the general formula (11) may be prepared under Mitsunobu conditions by reacting a compound of the general formula (2) with a compound of the general formula (10b), wherein R_{d} is C₁₋₄ alkyl, using a phosphine, such as triphenyl phosphine, and an azoester, such as diethyl azodicarboxylate.

Similarly, compounds of the general formula (1) may be prepared by reacting a compound of general formula (10d) with a compound of the general formula (2) under Mitsunobu conditions using a phosphine, such as triphenyl phosphine, and an azoester, such as diethyl azodicarboxylate. Compounds of general formula (10d) may be prepared from a compound of general formula (10c) and an amine of general formula (5) using suitable activating reagents such as 1-hydroxybenzotriazole and *N-*(3-dimethylaminopropyl)-*N'*-ethyl-carbodiimide hydrochloride. Compounds (10b) and (10c) are either known compounds or may be made from known compounds.

In another method, the compounds of the general formula (1) may be prepared by reacting an acid halide of the general formula (13) with an amine of the general formula (5) in a suitable solvent, such as dichloromethane, in the presence of a tertiary amine, such as triethylamine, and an activating agent, such as 4-dimethylaminopyridine.

For example, as shown in Scheme 5, an acid chloride of the general formula (13) where Hal is C1 may be prepared by chlorinating a compound of the general formula (12) with a suitable chlorinating agent, such as oxalyl chloride, in a suitable solvent, such as dichloromethane, and in the presence of, for example, *N,N*-dimethylformamide. The compounds of the general formula (12) correspond to the compounds of general formula (11), wherein R_{d} is H.

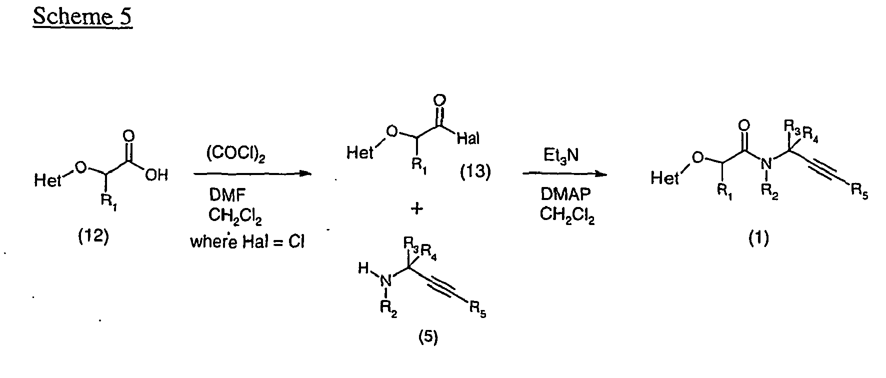

As shown in Scheme 6, compounds of the general formula (1), wherein R₅ is H, may be reacted under Sonogashira conditions with, for example, optionally substituted phenyl or thienyl chlorides, bromides, iodides or triflates to form substituted phenyl or thienyl compounds of general formula (1), wherein R₅ is an optionally substituted phenyl or thienyl group. A suitable palladium catalyst is tetrakis(triphenylphosphine)-palladium(0).

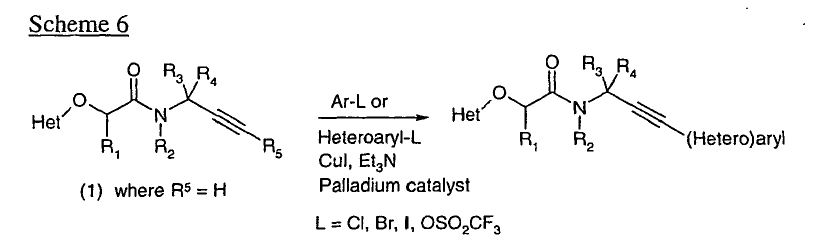

Compounds of the general formula (1) wherein R₁ is straight-chain C₁₋₄ alkoxy, such as compounds of the general formula (14) wherein R₆ is as defined above, may be prepared as shown in Scheme 7. Thus, esters of the formula (15) may be halogenated to give haloesters of the general formula (16), by treatment with a suitable halogenating agent, such as N-bromosuccinimide, in a suitable solvent such as carbon tetrachloride or acetonitrile, at between ambient temperature and the reflux temperature of the solvent. The haloesters of the general formula (16) can be reacted with an alkali metal compound M⁺OR₆, where M is suitably sodium or potassium in, for example, an alcohol R₆OH as solvent, at between 0°C and 40°C, preferably at ambient temperature, to give compounds of the general formula (17). Alternatively haloethers of general formula (18) can be reacted with a compound of the general formula (2), in which the OH group is in the 5- or 6-position of the Het ring system, in the presence of a base such as sodium hydride or potassium t-butoxide, in a suitable solvent, for example THF or DMF, at between 0°C and 60°C, preferably at ambient temperature, to give compounds of the general formula (17). The esters (17) can be hydrolysed to acids of the general formula (19), by treatment with an alkali metal hydroxide, such as sodium hydroxide, in an aqueous alcohol R₆OH, at between ambient temperature and reflux temperature of the solvent. A carboxylic acid of the general formula (19) can be condensed with an amine of the general formula (5) to give a compound of the general formula (14), where R₆ is as defined above, using suitable activating reagents such as 1-hydroxybenzotriazole and *N-*(3-dimethylaminopropyl)-*N*'-ethylcarbodiimide hydrochloride.

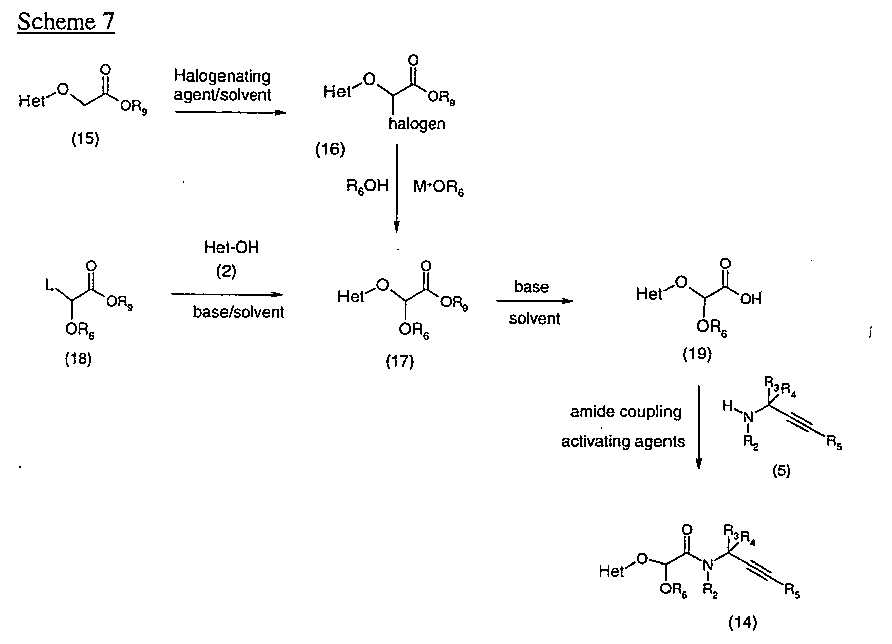

Compounds of the general formula (1), wherein R₁ is C₁₋₄ alkyl, C₃₋₄ alkenyl, C₃₋₄ alkynyl, or an alkoxyalkyl group where the total number of carbon atoms is 2 or 3, may be prepared as shown in Scheme 8. Thus, the substituted acetic acid (20) may be treated with at least two equivalents of a base, such as lithium di*iso*propylamide, in a suitable solvent such as tetrahydrofuran, at a temperature between -78°C and ambient temperature, with an alkylating agent such as R₁L to give carboxylic acids of the general formula (21) upon acidification.

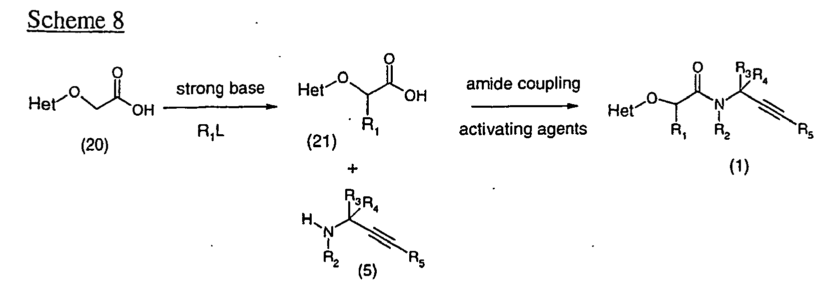

As shown in Scheme 9, compounds of the general formula (1), where R₁ is a C₃₋₄ alkenyl group, may be prepared from esters of the general formula (22), wherein R₇ and R₈ are as defined above. Esters of the general formula (22) are treated with a strong base, such as lithium *bis*(trimethylsilyl)amide, at between -78°C and ambient temperature, preferably at -78°C, and then reacted with a trialkylsilyl chloride R₃SiCl, such as trimethylsilyl chloride, or trialkylsilyl triflate R₃SiOSO₂CF₃, and allowed to warm to ambient temperature. The resultant acids of the general formula (23) obtained after hydrolysis can be condensed with amines of the general formula (5) to give the compounds of the general formula (24), using suitable activating reagents such as 1-hydroxybenzotriazole and *N*-(3-dimethylaminopropyl)-*N*'-ethyl- carbodiimide hydrochloride.

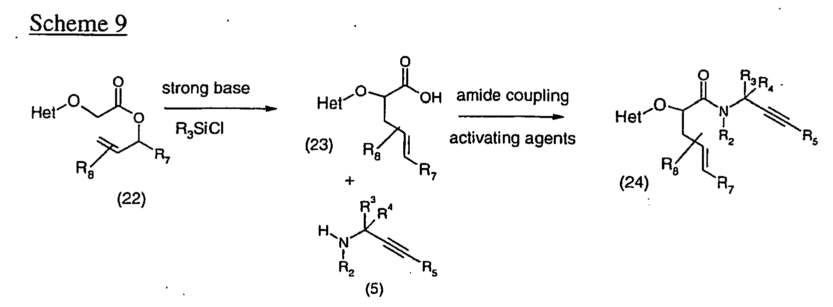

Other compounds of the invention may be prepared by transforming the substituents in the compounds of the general formula (1) using known procedures e.g. by the alkylation of compounds of the general formula (1), wherein R₂ is H or R₅ is H.

Typical routes for the construction of suitable Het are detailed in Comprehensive Heterocyclic Chemistry (Eds. in chief A. R. Katritzky, C. W. Rees, E. F. V. Schriven) Elsevier Science Ltd. (1999). Examples of Het-OH are commercially available, known in the literature or may be made by transformation of known heterocycles.

The compounds of formula (1) are active fungicides and may be used to control one or more of the following pathogens: *Pyricularia oryzae (Magnaporthe grisea)* on rice and wheat and other *Pyricularia* spp. on other hosts; *Puccinia triticina* (or *recondita), Puccinia striiformis.* and other rusts on wheat, *Puccinia horde, Puccinia striiformis* and other rusts on barley, and rusts on other hosts (for example turf, rye, coffee, pears, apples, peanuts, sugar beet, vegetables and ornamental plants); *Erysiphe cichoracearum* on cucurbits (for example melon); *Blumeria* (or *Erysiphe*) *graminis* (powdery mildew) on barley, wheat, rye and turf and other powdery mildews on various hosts, such as *Sphaerotheca macularis* on hops, *Sphaerotheca fusca (Sphaerotheca fuliginea)* on cucurbits (for example cucumber), *Leveillula taurica* on tomatoes, aubergine and green pepper, *Podosphaera leucotricha* on apples and *Uncinula necator* on vines; *Cochliobolus* spp., *Helminthosporium* spp., *Drechslera* spp. (*Pyrerzophora* spp.), *Rhynchosporium* spp., *Mycosphaerella graminicola (Septoria tritici)* and *Phaeosphaeria nodorum (Stagonospora nodorum* or *Septoria nodorum), Pseudocercosporella herpotrichoides* and *Gaeumannomyces graminis* on cereals (for example wheat, barley, rye), turf and other hosts; *Cercospora arachidicola* and *Cercosporidium personatum* on peanuts and other *Cercospora* spp. on other hosts, for example sugar beet, bananas, soya beans and rice; *Botrytis cinerea* (grey mould) on tomatoes, strawberries, vegetables, vines and other hosts and other *Botrytis* spp. on other hosts; *Alternaria* spp. on vegetables (for example carrots), oil-seed rape, apples, tomatoes, potatoes, cereals (for example wheat) and other hosts; *Venturia* spp. (including *Venturia inaequalis* (scab)) on apples, pears, stone fruit, tree nuts and other hosts; *Cladosporium* spp. on a range of hosts including cereals (for example wheat) and tomatoes; *Monilinia* spp. on stone fruit, tree nuts and other hosts; *Didymella* spp. on tomatoes, turf, wheat, cucurbits and other hosts; *Phoma* spp. on oil-seed rape, turf, rice, potatoes, wheat and other hosts; *Aspergillus* spp. and *Aureobasidium* spp. on wheat, lumber and other hosts; *Ascochyta* spp. on peas, wheat, barley and other hosts; *Stemphylium* spp. (*Pleospora* spp.) on apples, pears, onions and other hosts; summer diseases (for example bitter rot *(Glomerella cingulata),* black rot or frogeye leaf spot *(Botryosphaeria obtusa),* Brooks fruit spot *(Mycosphaerella pomi),* Cedar apple rust *(Gymnosporangium juniperi-virginianae),* sooty blotch *(Gloeodes pomigena),* flyspeck *(Schizothyrium pomi)* and white rot *(Botryosphaeria dothidea))* on apples and pears; *Plasmopara viticola* on vines; other downy mildews, such as *Bremia lactucae* on lettuce, *Peronospora* spp. on soybeans, tobacco, onions and other hosts, *Pseudoperonospora humuli* on hops and *Pseudoperonospora cubensis* on cucurbits; *Pythium* spp. (including *Pythium ultimum)* on turf and other hosts; *Phytophthora infestans* on potatoes and tomatoes and other *Phytophthora* spp. on vegetables, strawberries, avocado, pepper, ornamentals, tobacco, cocoa and other hosts; *Thanatephorus cucumeris* on rice and turf and other *Rhizoctonia* spp. on various hosts such as wheat and barley, peanuts, vegetables, cotton and turf; *Sclerotinia* spp. on turf, peanuts, potatoes, oil-seed rape and other hosts; *Sclerotium* spp. on turf, peanuts and other hosts; *Gibberella fujikuroi* on rice; *Colletotrichum* spp. on a range of hosts including turf, coffee and vegetables; *Laetisaria fuciformis* on turf; *Mycosphaerella* spp. on bananas, peanuts, citrus, pecans, papaya and other hosts; *Diaporthe* spp. on citrus, soybean, melon, pears, lupin and other hosts; *Elsinoe* spp. on citrus, vines, olives, pecans, roses and other hosts; *Verticillium* spp. on a range of hosts including hops, potatoes and tomatoes; *Pyrenopeziza* spp. on oil-seed rape and other hosts; *Oncobasidium theobromae* on cocoa causing vascular streak dieback; *Fusarium* spp., *Typhula* spp., *Microdochium nivale, Ustilago* spp., *Urocystis* spp., *Tilletia* spp. and *Claviceps purpurea* on a variety of hosts but particularly wheat, barley, turf and maize; *Ramularia* spp. on sugar beet, barley and other hosts; post-harvest diseases particularly of fruit (for example *Penicillium digitatum, Penicillium italicum* and *Trichoderma viride* on oranges, *Colletotrichum musae* and *Gloeosporium musarum* on bananas and *Botrytis cinerea* on grapes); other pathogens on vines, notably *Eutypa lata, Guignardia bidwellii, Phellinus igniarus, Phomopsis viticola, Pseudopeziza tracheiphila* and *Stereum hirsutum;* other pathogens on trees (for example *Lophodermium seditiosum)* or lumber, notably *Cephaloascus fragrans, Ceratocystis* spp., *Ophiostoma piceae, Penicillium* spp., *Trichoderma pseudokoningii, Trichoderma viride, Trichoderma harzianum, Aspergillus niger, Leptographium lindbergi* and *Aureobasidium pullulans;* and fungal vectors of viral diseases (for example *Polymyxa graminis* on cereals as the vector of barley yellow mosaic virus (BYMV) and *Polymyxa betae* on sugar beet as the vector of rhizomania).

The compounds of formula (I) show particularly good activity against the Oomycete class of pathogens such as *Phytophthora infestans, Plasmopara* species, e.g. *Plasmopara viticola* and *Pythium.* species e.g. *Pythium ultimum.*

A compound of formula (1) may move acropetally, basipetally or locally in plant tissue to be active against one or more fungi. Moreover, a compound of formula (1) may be volatile enough to be active in the vapour phase against one or more fungi on the plant.

The invention therefore provides a method of combating or controlling phytopathogenic fungi which comprises applying a fungicidally effective amount of a compound of formula (1), or a composition containing a compound of formula (1), to a plant, to a seed of a plant, to the locus of the plant or seed or to soil or any other plant growth medium, e.g. nutrient solution.

The term "plant" as used herein includes seedlings, bushes and trees. Furthermore, the fungicidal method of the invention includes protectant, curative, systemic, eradicant and antisporulant treatments.

The compounds of formula (1) are preferably used for agricultural, horticultural and turfgrass purposes in the form of a composition.

In order to apply a compound of formula (1) to a plant, to a seed of a plant, to the locus of the plant or seed or to soil or any other growth medium, a compound of formula (1) is usually formulated into a composition which includes, in addition to the compound of formula (1), a suitable inert diluent or carrier and, optionally, a surface active agent (SFA). SFAs are chemicals which are able to modify the properties of an interface (for example, liquid/solid, liquid/air or liquid/liquid interfaces) by lowering the interfacial tension and thereby leading to changes in other properties (for example dispersion, emulsification and wetting). It is preferred that all compositions (both solid and liquid formulations) comprise, by weight, 0.0001 to 95%, more preferably 1 to 85%, for example 5 to 60%, of a compound of formula (1). The composition is generally used for the control of fungi such that a compound of formula (1) is applied at a rate of from 0.1g to 10kg per hectare, preferably from 1g to 6kg per hectare, more preferably from 1g to 1kg per hectare.

When used in a seed dressing, a compound of formula (1) is used at a rate of 0.0001g to 10g (for example 0.001g or 0.05g), preferably 0.005g to 10g, more preferably 0.005g to 4g, per kilogram of seed.

In another aspect the present invention provides a fungicidal composition comprising a fungicidally effective amount of a compound of formula (1) and a suitable carrier or diluent therefore.

In a still further aspect the invention provides a method of combating and controlling fungi at a locus, which comprises treating the fungi, or the locus of the fungi with a fungicidally effective amount of a composition comprising a compound of formula (1).

The compositions can be chosen from a number of formulation types, including dustable powders (DP), soluble powders (SP), water soluble granules (SG), water dispersible granules (WG), wettable powders (WP), granules (GR) (slow or fast release), soluble concentrates (SL), oil miscible liquids (OL), ultra low volume liquids (UL), emulsifiable concentrates (EC), dispersible concentrates (DC), emulsions (both oil in water (EW) and water in oil (EO)), micro-emulsions (ME), suspension concentrates (SC), aerosols, fogging/smoke formulations, capsule suspensions (CS) and seed treatment formulations. The formulation type chosen in any instance will depend upon the particular purpose envisaged and the physical, chemical and biological properties of the compound of formula (1).

Dustable powders (DP) may be prepared by mixing a compound of formula (1) with one or more solid diluents (for example natural clays, kaolin, pyrophyllite, bentonite, alumina, montmorillonite, kieselguhr, chalk, diatomaceous earths, calcium phosphates, calcium and magnesium carbonates, sulphur, lime, flours, talc and other organic and inorganic solid carriers) and mechanically grinding the mixture to a fine powder.

Soluble powders (SP) may be prepared by mixing a compound of formula (1) with one or more water-soluble inorganic salts (such as sodium bicarbonate, sodium carbonate or magnesium sulphate) or one or more water-soluble organic solids (such as a polysaccharide) and, optionally, one or more wetting agents, one or more dispersing agents or a mixture of said agents to improve water dispersibility/solubility. The mixture is then ground to a fine powder. Similar compositions may also be granulated to form water soluble granules (SG).

Wettable powders (WP) may be prepared by mixing a compound of formula (1) with one or more solid diluents or carriers, one or more wetting agents and, preferably, one or more dispersing agents and, optionally, one or more suspending agents to facilitate the dispersion in liquids. The mixture is then ground to a fine powder. Similar compositions may also be granulated to form water dispersible granules (WG).

Granules (GR) may be formed either by granulating a mixture of a compound of formula (1) and one or more powdered solid diluents or carriers, or from pre-formed blank granules by absorbing a compound of formula (1) (or a solution thereof, in a suitable agent) in a porous granular material (such as pumice, attapulgite clays, fuller's earth, kieselguhr, diatomaceous earths or ground corn cobs) or by adsorbing a compound of formula (1) (or a solution thereof, in a suitable agent) on to a hard core material. (such as sands, silicates, mineral carbonates, sulphates or phosphates) and drying if necessary. Agents which are commonly used to aid absorption or adsorption include solvents (such as aliphatic and aromatic petroleum solvents, alcohols, ethers, ketones and esters) and sticking agents (such as polyvinyl acetates, polyvinyl alcohols, dextrins, sugars and vegetable oils). One or more other additives may also be included in granules (for example an emulsifying agent, wetting agent or dispersing agent).

Dispersible Concentrates (DC) may be prepared by dissolving a compound of formula (1) in water or an organic solvent, such as a ketone, alcohol or glycol ether. These solutions may contain a surface active agent (for example to improve water dilution or prevent crystallisation in a spray tank).

Emulsifiable concentrates (EC) or oil-in-water emulsions (EW) may be prepared by dissolving a compound of formula (1) in an organic solvent (optionally containing one or more wetting agents, one or more emulsifying agents or a mixture of said agents). Suitable organic solvents for use in ECs include aromatic hydrocarbons (such as alkylbenzenes or alkylnaphthalenes, exemplified by SOLVESSO 100, SOLVESSO 150 and SOLVESSO 200; SOLVESSO is a Registered Trade Mark), ketones (such as cyclohexanone or methylcyclohexanone), alcohols (such as benzyl alcohol, furfuryl alcohol or butanol), N-alkylpyrrolidones (such as *N*-methylpyrrolidone or *N-*octylpyrrolidone), dimethyl amides of fatty acids (such as C₈-C₁₀ fatty acid dimethylamide) and chlorinated hydrocarbons. An EC product may spontaneously emulsify on addition to water, to produce an emulsion with sufficient stability to allow spray application through appropriate equipment. Preparation of an EW involves obtaining a compound of formula (1) either as a liquid (if it is not a liquid at room temperature, it may be melted at a reasonable temperature, typically below 70°C) or in solution (by dissolving it in an appropriate solvent) and then emulsifying the resultant liquid or solution into water containing one or more SFAs, under high shear, to produce an emulsion. Suitable solvents for use in EWs include vegetable oils, chlorinated hydrocarbons (such as chlorobenzenes), aromatic solvents (such as alkylbenzenes or alkylnaphthalenes) and other appropriate organic solvents which have a low solubility in water.

Microemulsions (ME) may be prepared by mixing water with a blend of one or more solvents with one or more SFAs, to produce spontaneously a thermodynamically stable isotropic liquid formulation. A compound of formula (1) is present initially in either the water or the solvent/SFA blend. Suitable solvents for use in MEs include those hereinbefore described for use in in ECs or in EWs. An ME may be either an oil-in-water or a water-in-oil system (which system is present may be determined by conductivity, measurements) and may be suitable for mixing water-soluble and oil-soluble pesticides in the same formulation. An ME is suitable for dilution into water, either remaining as a microemulsion or forming a conventional oil-in-water emulsion.

Suspension concentrates (SC) may comprise aqueous or non-aqueous suspensions of finely divided insoluble solid particles of a compound of formula (1). SCs may be prepared by ball or bead milling the solid compound of formula (1) in a suitable medium, optionally with one or more dispersing agents, to produce a fine particle suspension of the compound. One or more wetting agents may be included in the composition and a suspending agent may be included to reduce the rate at which the particles settle. Alternatively, a compound of formula (1) may be dry milled and added to water, containing agents hereinbefore described, to produce the desired end product.

Aerosol formulations comprise a compound of formula (1) and a suitable propellant (for example *n*-butane). A compound of formula (1) may also be dissolved or dispersed in a suitable medium (for example water or a water miscible liquid, such as *n-*propanol) to provide compositions for use in non-pressurised, hand-actuated spray pumps.

A compound of formula (1) may be mixed in the dry state with a pyrotechnic mixture to form a composition suitable for generating, in an enclosed space, a smoke containing the compound.

Capsule suspensions (CS) may be prepared in a manner similar to the preparation of EW formulations but with an additional polymerisation stage such that an aqueous dispersion of oil droplets is obtained, in which each oil droplet is encapsulated by a polymeric shell and contains a compound of formula (1) and, optionally, a carrier or diluent therefor. The polymeric shell may be produced by either an interfacial polycondensation reaction or by a coacervation procedure. The compositions may provide for controlled release of the compound of formula (1) and they may be used for seed treatment. A compound of formula (1) may also be formulated in a biodegradable polymeric matrix to provide a slow, controlled release of the compound.

A composition may include one or more additives to improve the biological performance of the composition (for example by improving wetting, retention or distribution on surfaces; resistance to rain on treated surfaces; or uptake or mobility of a compound of formula (1)). Such additives include surface active agents, spray additives based on oils, for example certain mineral oils or natural plant oils (such as soy bean and rape seed oil), and blends of these with other bio-enhancing adjuvants (ingredients which may aid or modify the action of a compound of formula (1)).

A compound of formula (1) may also be formulated for use as a seed treatment, for example as a powder composition, including a powder for dry seed treatment (DS), a water soluble powder (SS) or a water dispersible powder for slurry treatment (WS), or as a liquid composition, including a flowable concentrate (FS), a solution (LS) or a capsule suspension (CS). The preparations of DS, SS, WS, FS and LS compositions are very similar to those of, respectively, DP, SP, WP, SC and DC compositions described above. Compositions for treating seed may include an agent for assisting the adhesion of the composition to the seed (for example a mineral oil or a film-forming barrier).

Wetting agents, dispersing agents and emulsifying agents may be SFAs of the cationic, anionic, amphoteric or non-ionic type.

Suitable SFAs of the cationic type include quaternary, ammonium compounds (for example cetyltrimethyl ammonium bromide), imidazolines and amine salts.

Suitable anionic SFAs include alkali metals salts of fatty acids, salts of aliphatic monoesters of sulphuric acid (for example sodium lauryl sulphate), salts of sulphonated aromatic compounds (for example sodium dodecylbenzenesulphonate, calcium dodecylbenzenesulphonate, butylnaphthalene sulphonate and mixtures of sodium di-isopropyl- and tri-*iso*propyl-naphthalene sulphonates), ether sulphates, alcohol ether sulphates (for example sodium laureth-3-sulphate), ether carboxylates (for example sodium laureth-3-carboxylate), phosphate esters (products from the reaction between one or more fatty alcohols and phosphoric acid (predominately mono-esters) or phosphorus pentoxide (predominately di-esters), for example the reaction between lauryl alcohol and tetraphosphoric acid; additionally these products may be ethoxylated), sulphosuccinamates, paraffin or olefine sulphonates, taurates and lignosulphonates.

Suitable SFAs of the amphoteric type include betaines, propionates and glycinates.

Suitable SFAs of the non-ionic type include condensation products of alkylene oxides, such as ethylene oxide, propylene oxide, butylene oxide or mixtures thereof, with fatty alcohols (such as oleyl alcohol or cetyl alcohol) or with alkylphenols (such as octylphenol, nonylphenol or octylcresol); partial esters derived from long chain fatty acids or hexitol anhydrides; condensation products of said partial esters with ethylene oxide; block polymers (comprising ethylene oxide and propylene oxide); alkanolamides; simple esters (for example fatty acid polyethylene glycol esters); amine oxides (for example lauryl dimethyl amine oxide); and lecithins.

Suitable suspending agents include hydrophilic colloids (such as polysaccharides, polyvinylpyrrolidone or sodium carboxymethylcellulose) and swelling clays (such as bentonite or attapulgite).

A compound of formula (1) may be applied by any of the known means of applying fungicidal compounds. For example, it may be applied, formulated or unformulated, to any part of the plant, including the foliage, stems, branches or roots, to the seed before it is planted or to other media in which plants are growing or are to be planted (such as soil surrounding the roots, the soil generally, paddy water or hydroponic culture systems), directly or it may be sprayed on, dusted on, applied by dipping, applied as a cream or paste formulation, applied as a vapour or applied through distribution or incorporation of a composition (such as a granular composition or a composition packed in a water-soluble bag) in soil or an aqueous environment.

A compound of formula (1) may also be injected into plants or sprayed onto vegetation using electrodynamic spraying techniques or other low volume methods, or applied by land or aerial irrigation systems.

Compositions for use as aqueous preparations (aqueous solutions or dispersions) are generally supplied in the form of a concentrate containing a high proportion of the active ingredient, the concentrate being added to water before use. These concentrates, which may include DCs, SCs, ECs, EWs, MEs SGs, SPs, WPs, WGs and CSs, are often required to withstand storage for prolonged periods and, after such storage, to be capable of addition to water to form aqueous preparations which remain homogeneous for a sufficient time to enable them to be applied by conventional spray equipment. Such aqueous preparations may contain varying amounts of a compound of formula (1) (for example 0.0001 to 10%, by weight) depending upon the purpose for which they are to be used.

A compound of formula (1) may be used in mixtures with fertilisers (for example nitrogen-, potassium- or phosphorus-containing fertilisers). Suitable formulation types include granules of fertiliser. The mixtures suitably contain up to 25% by weight of the compound of formula (1).

The invention therefore also provides a fertiliser composition comprising a fertiliser and a compound of formula (1).

The compositions of this invention may contain other compounds having biological activity, for example micronutrients or compounds having similar or complementary fungicidal activity or which possess plant growth regulating, herbicidal, insecticidal, nematicidal or acaricidal activity.

By including another fungicide, the resulting composition may have a broader spectrum of activity or a greater level of intrinsic activity than the compound of formula (1) alone. Further the other fungicide may have a synergistic effect on the fungicidal activity of the compound of formula (1).

The compound of formula (1) may be the sole active ingredient of the composition or it may be admixed with one or more additional active ingredients such as a pesticide, fungicide, synergist, herbicide or plant growth regulator where appropriate. An additional active ingredient may: provide a composition having a broader spectrum of activity or increased persistence at a locus; synergise the activity or complement the activity (for example by increasing the speed of effect or overcoming repellency) of the compound of formula (1); or help to overcome or prevent the development of resistance to individual components. The particular additional active ingredient will depend upon the intended utility of the composition.

Examples of fungicidal compounds which may be included in the composition of the invention are AC 382042 (*N*-(1-cyan-1,2-dimethylpropyl)-2-(2,4-dichlorophenoxy) propionamide), acibenzolar-S-methyl, alanycarb, aldimorph, anilazine, azaconazole, azafenidin, azoxystrobin, benalaxyl, benomyl, benthiavalicarb, biloxazol, bitertanol, blasticidin S, boscalid (new name for nicobifen), bromuconazole, bupirimate, captafol, captan; carbendazim, carbendazim chlorhydrate, carboxin, carpropamid, carvone, CGA 41396, CGA 41397, chinomethionate, chlorbenzthiazone, chlorothalonil, chlorozolinate, clozylacon, copper containing compounds such as copper oxychloride, copper oxyquinolate, copper sulphate, copper tallate, and Bordeaux mixture, cyamidazosulfamid, cyazofamid (IKF-916), cyflufenamid, cymoxanil, cyproconazole, cyprodinil, debacarb, di-2-pyridyl disulphide 1,1'-dioxide, dichlofluanid, diclocymet, diclomezine, dicloran, diethofencarb, difenoconazole, difenzoquat, diflumetorim, *O,O*-di-iso-propyl-*S*-benzyl thiophosphate, dimefluazole, dimetconazole, dimethirimol, dimethomorph, dimoxystrobin, diniconazole, dinocap, dithianon, dodecyl dimethyl ammonium chloride, dodemorph, dodine, doguadine, edifenphos, epoxiconazole, ethaboxam, ethirimol, ethyl (Z)-*N*-benzyl-*N*([methyl(methyl-thioethylideneaminooxycarbonyl)amino]thio)-β-alaninate, etridiazole, famoxadone, fenamidone, fenarimol, fenbuconazole, fenfuram, fenhexamid, fenoxanil (AC 382042), fenpiclonil, fenpropidin, fenpropimorph, fentin acetate, fentin hydroxide, ferbam, ferimzone, fluazinam, fludioxonil, flumetover, flumorph, fluoroimide, fluoxastrobin, fluquinconazole, flusilazole, flusulfamide, flutolanil, flutriafol, folpet, fosetyl-aluminium, fuberidazole, furalaxyl, furametpyr, guazatine, hexaconazole, hydroxyisoxazole, hymexazole, imazalil, imibenconazole, iminoctadine, iminoctadine triacetate, ipconazole, iprobenfos, iprodione, iprovalicarb, isopropanyl butyl carbamate, isoprothiolane, kasugamycin, kresoxim-methyl, LY186054, LY211795, LY 248908, mancozeb, maneb, mefenoxam, mepanipyrim, mepronil, metalaxyl, metalaxyl M, metconazole, metiram, metiram-zinc, metominostrobin, metrafenone, MON65500 (*N*-allyl-4,5-dimethyl-2-trimethylsilylthiophene-3-carboxamide), myclobutanil, NTN0301, neoasozin, nickel dimethyldithiocarbamate, nitrothale-isopropyl, nuarimol, ofurace, organomercury compounds, orysastrobin, oxadixyl, oxasulfuron, oxolinic acid, oxpoconazole, oxycarboxin, pefurazoate, penconazole, pencycuron, phenazin oxide, phosphorus acids, phthalide, picoxystrobin, polyoxin D, polyram, probenazole, prochloraz, procymidone, propamocarb, propamocarb hydrochloride, propiconazole, propineb, propionic acid, proquinazid, prothioconazole, pyraclostrobin, pyrazophos, pyrifenox, pyrimethanil, pyroquilon, pyroxyfur, pyrrolnitrin, quaternary ammonium compounds, quinomethionate, quinoxyfen, quintozene, silthiofam (MON 65500), S-imazalil, simeconazole, sipconazole, sodium pentachlorophenate, spiroxamine, streptomycin, sulphur, tebuconazole, tecloftalam, tecnazene, tetraconazole, thiabendazole, thifluzamide, 2-(thiocyanomethylthio)benzothiazole, thiophanate-methyl, thiram, tiadinil, timibenconazole, tolclofos-methyl, tolylfluanid, triadimefon, triadimenol, triazbutil, triazoxide, tricyclazole, tridemorph, trifloxystrobin, triflumizole, triforine, triticonazole, validamycin A, vapam, vinclozolin, XRD-563, zineb, ziram, zoxamide and compounds of the formulae:

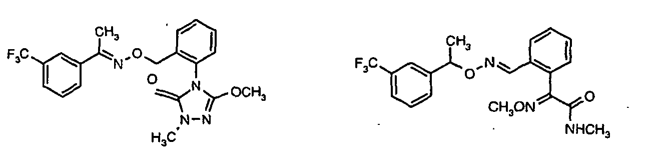

The compounds of formula (1) may be mixed with soil, peat or other rooting media for the protection of plants against seed-borne, soil-borne or foliar fungal diseases.

Some mixtures may comprise active ingredients which have significantly different physical, chemical or biological properties such that they do not easily lend themselves to the same conventional formulation type. In these circumstances other formulation types may be prepared. For example, where one active ingredient is a water insoluble solid and the other a water insoluble liquid, it may nevertheless be possible to disperse each active ingredient in the same continuous aqueous phase by dispersing the solid active ingredient as a suspension (using a preparation analogous to that of an SC) but dispersing the liquid active ingredient as an emulsion (using a preparation analogous to that of an EW). The resultant composition is a suspoemulsion (SE) formulation.

The invention is illustrated by the following Examples in which the following abbreviations are used:

| | |
|---|---|
| ml = millilitres | DMSO = dimethylsulphoxide |
| g = grammes | NMR = nuclear magnetic resonance |
| ppm = parts per million | HPLC = high performance liquid |
| M⁺ = mass ion | chromatography |
| M = molar | m.p. = melting point (uncorrected) |
| s = singlet | q = quartet |
| d = doublet | m = multiplet |
| t = triplet | bs = broad singlet |

### EXAMPLE 1

This Example illustrates the preparation of 2-(6-benzothiazolyloxy)-*N*-(4-methylpent-2-yn-4-yl) butyramide (Compound No. 2 of Table 1)

### Stage 1: Preparation of 2-bromo-N-(4-methylpent-2-yn-4-yl) butyramide

### Step 1: Preparation of 4-amino-4-methylpent-2-yne hydrochloride

3-Amino-3-methylbutyne (commercially available as 90% aqueous solution; 16.6g) was dissolved in dichloromethane (150ml), dried over sodium sulphate and filtered to give a solution containing 14.9g of amine. To the stirred solution of amine under an atmosphere of nitrogen at ambient temperature was added dry triethylamine (48.4ml). 1,2-*Bis*-(chlorodimethylsilyl)ethane (38.98g) in dichloromethane (100ml) was then added dropwise, maintaining the reaction temperature at 15°C by cooling. The mixture was stirred for 3 hours, the colourless solid, which had formed during the reaction, was filtered from solution and the filtrate was evaporated under reduced pressure to give a paste. The paste was extracted into hexane and refiltered. The filtrate was evaporated under reduced pressure and the oil obtained was distilled to give 1-(1,1-dimethyl-2-propynyl)-2,2,5,5-tetramethyl-1-aza-2,5-disilacylopentane, 21.5g, b.p. 41°C at 0.06 mm Hg pressure.
¹H NMR (CDCl₃) δ: 0.16(12H, s); 0.60(4H, s); 1.48(6H, s); 2.24(1H, s).

### Step 2

The product from Step 1 (13.0g) in dry tetrahydrofuran (140ml) was cooled to-70°C under an atmosphere of nitrogen with stirring and a solution of *n*-butyl lithium (23.1ml of 2.5M solution in hexanes) was added at -65 to -70°C during 5 minutes. The mixture was allowed to warm to -5°C and methyl iodide (3.93ml) was added dropwise over 10 minutes. The reaction mixture was allowed to warm to 10°C when an exothermic reaction occurred. The mixture was maintained at 20°C by cooling for 2 hours then evaporated under reduced pressure to a small volume. The residue was dissolved in hexane, filtered to remove the insoluble material and evaporated under reduced pressure to give 1-(1,1-dimethyl-2-butynyl)-2,2,5,5-tetramethyl-1-aza-2,5-disilacyclopentane as a yellow oil, 13.0g.
¹H NMR (CDCl₃) δ: 0.10(12H, s); 0.56(4H, s); 1.40(6H, s); 1.72(3H, s).

### Step 3

The product from Step 2 (13.0g) was added slowly to aqueous hydrochloric acid (35ml, 4M) at 0°C with stirring. The emulsion formed was stirred for 0.5 hours then taken to pH14 with aqueous sodium hydroxide (4M) while maintaining the reaction mixture at 0°C by cooling in ice. The aqueous mixture was extracted into dichloromethane (three times) and the extracts combined, dried over sodium sulphate and filtered. The filtrate was made acidic by adding an excess of a saturated solution of hydrogen chloride in 1,4-dioxan. The mixture was concentrated under reduced pressure until a colourless precipitate was formed. Hexane was added to the suspension and the solid was filtered from solution. The solid was washed with dry diethyl ether and placed under vacuum to remove any residual solvents to give the required product as a colourless solid, 5.0g.
¹H NMR (*d*₆-DMSO) δ: 1.74(6H, s); 1.82(3H, s); 8.74(3H, bs).

### Step 4: The preparation of 2-bromo-N-(4-methylpent-2-yn-4-yl) butyramide

The product from Step 3 (5.0g) was dissolved in dry dichloromethane (200ml), cooled to 3°C with stirring then 2-bromobutyryl bromide (6.25g) was added followed by dropwise addition of dry triethylamine (10.93ml), maintaining the reaction at 5°C. The suspension, which had formed during the reaction, was stirred at ambient temperature for 1 hour then water was added. The organic phase was separated, washed with water, dried over magnesium sulphate then evaporated under reduced pressure. The residue was fractionated by chromatography (silica; hexane / diethyl ether, 3:1 by volume) to give the required product, 5.2g, as a colourless solid, mp 79-81°C.
¹H NMR (CDCl₃) δ: 1.04(3H, t); 1.64(6H, s); 1.84(3H, s); 2.04-2.18(2H, m); 4.20-4.24(1H, m); 6.46 (1H, bs).

### Stage 2: The preparation of 6-hydroxybenzothiazole

### Step 1: Preparation of 6-methoxybenzothiazole

2-Amino-6-methoxybenzothiazole (9.0g, commercially available) in dry *N,N* dimethylformamide (10ml) was added dropwise over 35 minutes to a stirred solution of *tert*-butyl nitrite (9.9ml) in *N,N-*dimethylformamide (40ml) at 65°C. The temperature of the mixture was kept higher than 73°C during the addition. On complete addition of the solution of the benzothiazole, the dark red solution was stirred for an additional 15 minutes, cooled to ambient temperature then poured into dilute hydrochloric acid (200ml) and diluted with brine. The dark red suspension was extracted with diethyl ether and the solid filtered then washed with further water and diethyl ether. The diethyl ether extracts were combined and the aqueous fraction re-extracted with ethyl acetate. The organic fractions were combined, washed with water, dried over magnesium sulphate then evaporated under reduced pressure to give a brown solid. The solid was fractionated by chromatography (silica; hexane / ethyl acetate, 4:1 by volume) to give 6-methoxybenzothiazole, 2.1g, as a colourless solid.
¹H NMR (CDCl₃) δ: 3.89(3H, s); 7.12(1H, dd); 7.40(1H, d); 8.01(1H, d); 8.82(1H, s).

### Step 2: Preparation of 6-hydroxybenzothiazole

6-Methoxybenzothiazole (1.2g) in hydrobromic acid (10ml, 48%) was heated at 120°C with stirring for 6 hours then stored at ambient temperature for 2 days. The hot, pale yellow solution produced a suspension on cooling. The suspension was dissolved by the addition of water then the solution was adjusted to pH 6 by addition of sodium hydrogen carbonate and the solid which precipitated was filtered from solution, washed with water and sucked to dryness. The solid was dissolved in ethyl acetate, dried over magnesium sulphate and evaporated under reduced pressure to give 6-hydroxybenzothiazole, 1.05g, as a colourless solid.
¹HNMR (CDCl₃) δ: 7.07(1H, dd); 7.91(1H, d); 8.76(1H, d); 9.18(1H, s).
In a similar procedure, 6-methoxy-2-methylbenzothiazole (commercially available) was converted to 6-hydroxy-2-methylbenzothiazole, colourless solid.
¹H NMR (CDCl₃) δ: 2.80(3H, s); 6.99(1H, d); 7.28(1H, d); 7.32(1H, bs); 7.77(1H, d).

### Stage 3:

6-Hydroxybenzothiazole (0.151g) and 2-bromo-*N*-(4-methylpent-2-yn-4-yl) butyramide (0.246g) were stirred in dry *N,N*-dimethylformamide (2ml) containing anhydrous potassium carbonate (0.207g) and heated to 90°C for 6 hours. The mixture was cooled to ambient temperature, stored for 18 hours then taken to pH 7 with dilute hydrochloric acid. The suspension was diluted with water, extracted with diethyl ether and the extract was washed with water, aqueous sodium hydroxide then water and dried over magnesium sulphate. The dried extract was absorbed onto silica gel and this added to a column of silica gel and then fractionated by chromatography (silica; hexane / ethyl acetate, 1:1 by volume) to give the required product, 0.306g, as a colourless gum.
¹H NMR (CDCl₃) δ:1.07(3H, t); 1.59(3H, s); 1.60(3H, s); 1.79(3H, s); 1.95-2.04(2H, m); 4.47 (1H, t); 6.44 (1H, s); 7.17 (1H, dd); 7.43 (1H, m); 8.04(1H, d); 8.88(1H, s).

In a similar procedure, 6-hydroxy-2-methylbenzothiazole was reacted with 2-bromo-*N-*(4-methylpent-2-yn-4-yl) butyramide to give 2-(2-methylbenzothiazolyl-6-oxy)-*N*-(4-methylpent-2-yn-4-yl) butyramide (Compound No. 2 of Table 58), colourless solid, m.p. 84-87°C.
¹H NMR (CDCl₃) δ: 1.05(3H, t); 1.59(3H, s); 1.60(3H, s); 1.79(3H, s); 1.95-2.04(2H, m); 2.80(3H, s); 4.44(1H, t); 6.47(1H, s); 7.08(1H, dd); 7.32(1H, m); 7.84(1H, d); 8.88(1H, s).

In a similar procedure, 5-hydroxy-2-methylbenzothiazole was reacted with 2-bromo-*N-*(4-methylpent-2-yn-4-yl) butyramide to give 2-(2-methylbenzothiazolyl-5-oxy)-*N*-(4-methylpent-2-yn-4-yl) butyramide (Compound No. 2 of Table 62), gum.
¹H NMR (CDCl₃) δ: 1.05(3H, t); 1.59(3H, s); 1.60(3H, s); 1.82(3H, s); 2.00(2H, m); 2.83(3H, s); 4.50(1H, t); 6.50(1H, bs); 7.02(1H, dd); 7.49(1H, d); 7.70(1H, d).

### EXAMPLE 2

This Example illustrates the preparation of 2-(6-benzothiazolyloxy)- 3-methoxy-*N*-(4-methylpent-2-yn-4-yl)propionamide (Compound No. 2 of Table 11)

### Stage 1: The preparation of 2-bromo-3-methoxy-N-(4-methylpent-2-yn-4-yl)-propionamide

### Step 1: Preparation of methyl 2-bromo-3-methoxypropionate

Methyl 2,3-dibromopropionate (21.9g) and trimethylamine *N*-oxide (0.1g) in methanol (8ml) were cooled to -5°C with stirring under an atmosphere of nitrogen. A methanolic solution of sodium methoxide, freshly prepared from sodium (2.25g) and methanol (24ml), was added dropwise over 15 minutes to the mixture, which was maintained below 0°C by cooling. On completion of addition, the mixture was stirred for a further 30 minutes and acetic acid (1ml) was added followed by diethyl ether (100ml). The mixture was filtered to remove insoluble salts and the filtrate evaporated under reduced pressure to give an oil, which was re-dissolved in a small volume of diethyl ether and re-filtered. The filtrate was evaporated under reduced pressure to give the required product (17.4g) as a pale yellow oil.
¹H NMR (CDCl₃) δ: 3.41(3H, s); 3.74(1H, dd); 3.82(3H, s); 3.92(1H, dd); 4.34(1H, dd).

### Step 2: Preparation of 2-bromo-3-methoxypropionic acid.

Methyl 2-bromo-3-methoxypropionate (1.00g) in tetrahydrofuran (8ml) was stirred at 10°C and lithium hydroxide monohydrate (0.21g) in water (1.5ml) was added dropwise. On complete addition, the mixture was stirred for 1.5 hours. The colourless solution was evaporated under reduced pressure to a small volume and the aqueous solution was taken to pH 3 with dilute sulphuric acid. The mixture was extracted with diethyl ether (50ml) and the organic phase separated, washed with brine, dried over magnesium sulphate then evaporated under reduced pressure to give the required product (0.6g) as a colourless liquid.
¹H NMR (CDCl₃) δ: 3.45(3H, s); 3.78(1H, m); 3.92(1H, m); 4.38(1H, m); 6.65(1H, bs).

### Step 3: Preparation of 2-bromo-N-(4-methylpent-2-yn-4-yl) 3-methoxypropionamide.

2-Bromo-3-methoxypropionic acid (0.366g) was dissolved in dry dichloromethane (4ml) containing dry *N,N*-dimethylformamide (0.05ml) with stirring and oxalyl chloride (0.254g) was added. The mixture was stirred at ambient temperature for 2 hours then evaporated under reduced pressure to give 2-bromo-3-methoxypropionic acid chloride (C=O, ν 1780cms⁻¹). The acid chloride was dissolved in dry dichloromethane (6ml) and 4-amino-4-methylpent-2-yne hydrochloride (0.267g) was added. The mixture was cooled to 3°C and triethylamine (0.404g) was added dropwise, while keeping the reaction temperature between 0-5°C. The suspension that had formed was stirred at ambient temperature for 1 hour, diluted with further dichloromethane and washed with hydrochloric acid (2M). The organic phase was separated, dried over magnesium sulfate and evaporated under reduced pressure to give a gum. The gum was fractionated by chromatography (silica: hexane / ethyl acetate, 3:2 by volume) to give the required product (0.300g) as a colourless solid.
¹H NMR (CDCl₃) δ: 1.63(6H, s); 1.82(3H, s); 3.44(3H, s); 3.88(2H, m); 4.32(1H, m); 6.62(1H, s).

### Stage 2

6-Hydroxybenzothiazole (0.151g) and 2-bromo-3-methoxy-*N*-(4-methylpent-2-yn-4-yl) propionamide (0.262g) were stirred in dry *N,N-*dimethylformamide (2ml) containing anhydrous potassium carbonate (0.207g) and heated to 90°C for 5 hours. The mixture was cooled to ambient temperature, stored for 18 hours then taken to pH 7 with dilute hydrochloric acid. The suspension was diluted with water, extracted with diethyl ether and the extract was washed with water, aqueous sodium hydroxide then water and dried over magnesium sulphate. The dried extract was absorbed onto silica gel and this added to a column of silica gel and then fractionated by chromatography (silica; hexane / ethyl acetate, 1:1 by volume) to give the required product, 0.24g, as a colourless gum.
¹H NMR (CDCl₃) δ: 1.60(3H, s); 1.61(3H, s); 1.79(3H, s); 3.43(3H, s); 3.84-3.93(2H, m); 4.67(1H, t); 6.51(1H, s); 7.21(1H, dd); 7.50(1H, m); 8.05(1H, d); 8.88(1H, s).

### EXAMPLE 3

This Example illustrates the preparation of 2-(6-benzoxazolyloxy)-*N*-(4-methylpent-2-yn-4-yl) butyramide (Compound No. 2 of Table 39)

### Stage 1: Preparation of 6-hydroxybenzoxazole (based on a procedure described in US Patent No 6,130,217; preparation 38)

4-Amino-1,3-dihydroxybenzene hydrochloride (5.1g, commercially available) and triethyl orthoformate (7.0g) containing concentrated sulphuric acid (0.2g) were stirred and heated to boiling point allowing the ethanol that was produced as the reaction proceeded to distil from the reaction mixture. When no further distillate was generated, the dark tar that was produced on cooling the mixture was cooled to give a solid that was partitioned between water and ethyl acetate. The mixture was filtered to remove insoluble material and the two phases were separated. The aqueous phase was extracted with ethyl acetate and the organic fractions were combined, washed with brine then dried over magnesium sulphate. The solvent was evaporated under reduced pressure to leave a red oil that was fractionated by chromatography (silica; hexane / ethyl acetate) to give 6-hydroxybenzoxazole (0.3g).
¹H NMR (CDCl₃) δ: 6.92(1H, dd); 7.07(1H, d); 7.57(1H, d); 7.95(1H, s); 9.01(1H, s).

### Stage 2

6-Hydroxybenzoxazole (0.29g) and 2-bromo-*N*-(4-methylpent-2-yn-4-yl) butyramide (0.517g) were stirred in dry *N,N*-dimethylformamide (5ml) containing anhydrous potassium carbonate (0.414g) and heated to 80°C for 6 hours. The mixture was cooled to ambient temperature, stored for 18 hours then diluted with aqueous sodium hydroxide (2M). The emulsion produced was extracted with diethyl ether and the organic extract was washed with water, dried over magnesium sulphate then evaporated under reduced pressure to give a pale brown gum. The gum was absorbed onto silica gel, added to a column of silica gel and fractionated by chromatography (silica; hexane / ethyl acetate) to give a pink gum that solidified on triturating with a small volume of hexane/ diethyl ether to give the required product as a solid, 0.416g, m.p. 95-97 °C.
¹H NMR (CDCl₃) δ: 1.04(3H, t); 1.59(3H, s); 1.60(3H, s); 1.79(3H, s); 1.96-2.05(2H, m); 4.47(1H, t); 6.46(1H, s); 7.02(1H, dd); 7.13(1H, m); 7.69(1H, d); 8.02(1H, s).

In a similar procedure, 6-hydroxy-2-methylbenzoxazole (preparation described in *Synthesis* (**1982**), *1*, 68-69.) was reacted with 2-bromo-*N*-(4-methylpent-2-yn-4-yl) butyramide to give 2-(2-methylbenzoxazolyl-6-oxy)-N-(4-methylpent-2-yn-4-yl) butyramide, (Compound No 2 of Table 61) colourless gum.
¹H NMR (CDCl₃) δ: 1.04(3H, t); 1.59(3H, s); 1.60(3H, s); 1.79(3H, s); 1.93-2.04(2H, m); 2.61(3H, s); 4.42(1H, t); 6.48(1H, s); 6.93(1H, dd); 7.03(1H, m); 7.53(1H, d).

### EXAMPLE 4

This Example illustrates the fungicidal properties of compounds of formula (1).

The compounds were tested in a leaf disk assay, with methods described below. The test compounds were dissolved in DMSO and diluted into water to 200 ppm, 60 ppm and 20 ppm.
*Erysiphe graminis f.sp. hordei* (barley powdery mildew): Barley leaf segments were placed on agar in a 24-well plate and sprayed with a solution of the test compound. After allowing to dry completely, for between 12 and 24 hours, the leaf disks were inoculated with a spore suspension of the fungus. After appropriate incubation the activity of a compound was assessed four days after inoculation as preventive fungicidal activity.
*Erysiphe graminis f.sp. tritici* (wheat powdery mildew): Wheat leaf segments were placed on agar in a 24-well plate and sprayed with a solution of the test compound. After allowing to dry completely, for between 12 and 24 hours, the leaf disks were inoculated with a spore suspension of the fungus. After appropriate incubation the activity of a compound was assessed four days after inoculation as preventive fungicidal activity.
*Puccinia recondita f.sp. tritici* (wheat brown rust): Wheat leaf segments were placed on agar in a 24-well plate and sprayed with a solution of the test compound. After allowing to dry completely, for between 12 and 24 hours, the leaf disks were inoculated with a spore suspension of the fungus. After appropriate incubation the activity of a compound was assessed nine days after inoculation as preventive fungicidal activity.
*Septoria nodorum* (wheat glume blotch): Wheat leaf segments were placed on agar in a 24-well plate and sprayed with a solution of the test compound. After allowing to dry completely, for between 12 and 24 hours, the leaf disks were inoculated with a spore suspension of the fungus. After appropriate incubation the activity of a compound was assessed four days after inoculation as preventive fungicidal activity.
*Pyrenophora teres* (barley net blotch): Barley leaf segments were placed on agar in a 24-well plate and sprayed with a solution of the test compound. After allowing to dry completely, for between 12 and 24 hours, the leaf disks were inoculated with a spore suspension of the fungus. After appropriate incubation the activity of a compound was assessed four days after inoculation as preventive fungicidal activity.
*Pyricularia oryzae* (rice blast): Rice leaf segments were placed on agar in a 24-well plate and sprayed with a solution of the test compound. After allowing to dry completely, for between 12 and 24 hours, the leaf disks were inoculated with a spore suspension of the fungus. After appropriate incubation the activity of a compound was assessed four days after inoculation as preventive fungicidal activity.
*Botrytis cinerea* (grey mould): Bean leaf disks were placed on agar in a 24-well plate and sprayed with a solution of the test compound. After allowing to dry completely, for between 12 and 24 hours, the leaf disks were inoculated with a spore suspension of the fungus. After appropriate incubation the activity of a compound was assessed four days after inoculation as preventive fungicidal activity.
*Phytophthora infestans* (late blight of potato on tomato): Tomato leaf disks were placed on water agar in a 24-well plate and sprayed with a solution of the test compound. After allowing to dry completely, for between 12 and 24 hours, the leaf disks were inoculated with a spore suspension of the fungus. After appropriate incubation the activity of a compound was assessed four days after inoculation as preventive fungicidal activity.
*Plasmopara viticola* (downy mildew of grapevine): Grapevine leaf disks were placed on agar in a 24-well plate and sprayed a solution of the test compound. After allowing to dry completely, for between 12 and 24 hours, the leaf disks were inoculated with a spore suspension of the fungus. After appropriate incubation the activity of a compound was assessed seven days after inoculation as preventive fungicidal activity.
*Pythium ultimum* (Damping off): Mycelial fragments of the fungus, prepared from a fresh liquid culture, were mixed into potato dextrose broth. A solution of the test compound in dimethyl sulphoxide was diluted with water to 20 ppm then placed into a 96-well microtiter plate and the nutrient broth containing the fungal spores was added. The test plate was incubated at 24°C and the inhibition of growth was determined photometrically after 48 hours.
The following Compounds (number of compound first, followed by table number in brackets) gave at least 60% control of the following fungal infection at 200ppm:
*Erysiphe grainis f.sp. hordei:* 2(39), 2(58).
*Erysiphe grainis f.sp. tritici:* 2(58), 2(61), 2(62).
*Phytophthora infestans:* 2(1),2(11).
*Plasmopara viticola:* 2(1), 2(11), 2(61).
The following Compounds (number of compound first, followed by table number in brackets) gave at least 60% control of the following fungal infection at 20ppm:
*Pythium ultimum:* 2(1),2(39).

## Claims

1. A compound of the general formula (1): 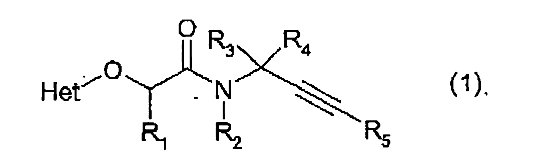 wherein Het is
5-or 6-benzothiazolyl optionally bearing a 2-C substituent, 5-(2,1-benzisothiazolyl) optionally bearing a 3-C substituent, 6-benzoxazolyl optionally bearing a 2-C substituent, 5-(2,1-benzisoxazolyl) optionally bearing a 3-C substituent, 6-(1*H*-benzimidazolyl) optionally bearing a 2-C substituent and optionally bearing a *N*-C₁₋₄ alkyl substituent, 5-(1*H-*indazolyl) optionally bearing a 3-C substituent and optionally bearing a *N*-C₁₋₄ alkyl substituent, 6-(1,2,3-benzothiadiazolyl) or 6-(1,2,3-benzoxadiazolyl), wherein any of the foregoing optional substituents are selected from halo, C₁₋₄ alkyl, C₁₋₄ alkoxy, C₁₋₄ alkylthio, C₁₋₄ alkylsulphinyl, C₁₋₄ alkylsulphonyl, halo-(C₁₋₄)alkyl or halo(C₁₋₄)alkoxy, halo(C₁₋₄)alkylthio, halo(C₁₋₄)alkylsulphinyl, halo(C₁₋₄)alkylsulphonyl or mono- or di-(C₁₋₄) alkylamino; R₁ is methyl, ethyl, *n-*propyl, 2,2,2-trifluorometyl cyanomethyl, acetylmethyl, methoxycarbonyl-methyl, methoxycarbonylethyl, hydroxymethyl, hydroxyethyl, methoxymethyl, methylthiomethyl, ethoxymethyl, 2-methoxyethyl, methoxy, ethoxy, *n*-propoxy or *n*-butoxy; R₂ is H; R₃ and R₄ are both methyl; and R₅ is methyl, hydroxymethyl, methoxymethyl, 1-methoxyethyl, 3-cyano-n-propyl or *tert*-butyldimethylsiloxymethyl.

2. A fungicidal composition comprising a fungicidally effective amount of a compound of formula (1) and a suitable carrier or diluent therefor.

3. A method of combating or controlling phytopathogenic fungi which comprises applying a fungicidally effective amount of a compound of formula (1) as defined in claim 1 or a composition according to claim 2 to a plant, to a seed of a plant, to the locus of the plant or seed or to soil or any other plant growth medium.

## Patentansprüche

1. Verbindungen der allgemeinen Formel (1): 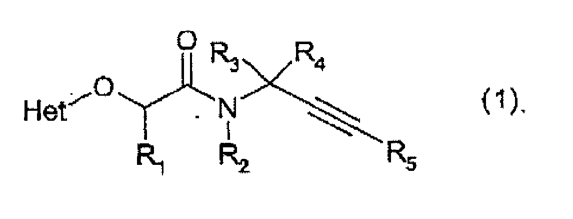 wobei Het für 5- oder 6-Benzothiazolyl, das gegebenenfalls einen 2-C-Substituenten trägt, 5-(2,1-Benzisothiazolyl), das gegebenenfalls einen 3-C-Substituenten trägt, 6-Benzoxazolyl, das gegebenenfalls einen 2-C-Substituenten trägt, 5-(2,1-Benzisoxazolyl), das gegebenenfalls einen 3-C-Substituenten trägt, 6-(1*H*-Benzimidazolyl), das gegebenenfalls einen 2-C-Substituenten trägt und gegebenenfalls einen *N*-C₁₋₄-Alkylsubstituenten trägt, 5-(1*H*-Indazolyl), das gegebenenfalls einen 3-C-Substituenten trägt und gegebenenfalls einen *N*-C₁₋₄-Alkylsubstituenten trägt, 6-(1,2,3-Benzothiadiazolyl) oder 6-(1,2,3-Benzoxadiazolyl) steht, wobei die obigen gegebenenfalls vorhandenen Substituenten ausgewählt sind aus Halogen, C₁₋₄-Alkyl, C₁₋₄-Alkoxy, C₁₋₄-Alkylthio, C₁₋₄-Alkylsulfinyl, C₁₋₄-Alkylsulfonyl, Halogen-(C₁₋₄)-alkyl, oder Halogen-(C₁₋₄)-alkoxy, Halogen-(C₁₋₄)-alkylthio, Halogen-(C₁₋₄)-alkylsulfinyl, Halogen-(C₁₋₄) -alkylsulfonyl oder Mono- oder Di-(C₁₋₄)-alkylamino; R₁ für Methyl, Ethyl, *n-*Propyl, 2,2,2-Trifluormethyl, Cyanomethyl, Acetylmethyl, Methoxycarbonylmethyl, Methoxycarbonylethyl, Hydroxymethyl, Hydroxyethyl, Methoxymethyl, Methylthiomethyl, Ethoxymethyl, 2-Methoxyethyl, Methoxy, Ethoxy, *n*-Propoxy oder *n-*Butoxy steht; R₂ für H steht; R₃ und R₄ beide für Methyl stehen und R₅ für Methyl, Hydroxymethyl, Methoxymethyl, 1-Methoxyethyl, 3-Cyano-*n*-propyl oder tert.-Butyldimethylsiloxymethyl steht.

2. Fungizide Zusammensetzung, enthaltend eine fungizid wirksame Menge einer Verbindung der Formel (1) und einen dafür geeigneten Träger oder ein dafür geeignetes Verdünnungsmittel.

3. Verfahren zur Bekämpfung bzw. Kontrolle phytopathogener Pilze, bei dem man eine fungizid wirksame Menge einer wie in Anspruch 1 definierten Verbindung der Formel (1) oder eine Zusammensetzung gemäß Anspruch 2 auf eine Pflanze, einen Pflanzensamen, auf die Stelle, an der sich die Pflanze oder der Samen befindet, oder auf Erdboden oder ein anderes Pflanzenwachstumsmedium aufbringt.

## Revendications

1. Composé de formule générale (1)_{:} 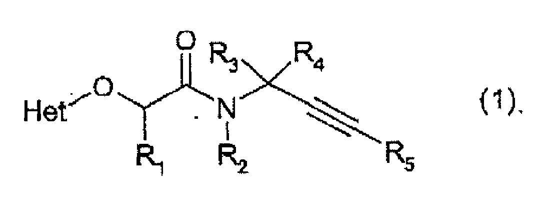 dans laquelle Het est 5- ou 6-benzothiazolyle portant éventuellement un substituant en 2-C, 5-(2,1-benzisothiazolyle) portant éventuellement un substituant en 3-C, 6-benzoxazolyle portant éventuellement un substituant en 2-C, 5-(2,1-benzisoxazolyle) portant éventuellement un substituant en 3-C, 6-(1*H*-benzimidazolyle) portant éventuellement un substituant en 2-C et portant éventuellement un substituant *N*-C₁₋₄ alkyle, 5-(1*H-*indazolyle) portant éventuellement un substituant en 3-C et portant éventuellement un substituant *N-*C₁₋₄ alkyle, 6-(1,2,3-benzothiadiazolyle) ou 6-(1,2,3-benzoxadiazolyle), des substituants quelconques parmi les substituants éventuels précédents étant choisis parmi halogéno, C₁₋₄ alkyle, C₁₋₄ alcoxy, C₁₋₄ alkylthio, C₁₋₄ alkylsulfinyle, C₁₋₄ alkylsulfonyle, halogéno (C₁₋₄) alkyle, ou halogéno (C₁₋₄) alcoxy, halogéno (C₁₋₄) alkylthio, halogéno (C₁₋₄) alkylsulfinyle, halogéno (C₁₋₄) alkylsulfonyle ou mono- ou di-(C₁₋₄) alkylamino; R₁ est méthyle, éthyle, *n-*propyle, 2,2,2-trifluorométhyle, cyanométhyle, acétylméthyle, méthoxycarbonylméthyle, méthoxycarbonyléthyle, hydroxyméthyle, hydroxyéthyle, méthoxyméthyle, méthylthiométhyle, éthoxyméthyle, 2-méthoxyéthyle, méthoxy, éthoxy, *n*-propoxy ou *n*-butoxy; R₂ est H ; R₃ et R₄ sont tous deux méthyle; e t R₅ est méthyle, hydroxyméthyle, méthoxyméthyle, 1-méthoxyéthyle, 3-cyano-*n*-propyle ou *tert-*butyldiméthylsiloxyméthyle.

2. Composition fongicide comprenant une quantité efficace du point de vue fongicide d'un composé de formule (1) et un support ou un diluant convenable pour celui-ci.

3. Méthode pour combattre ou lutter contre les champignons phytopathogènes qui comprend l'application d'une quantité efficace du point de vue fongicide d'un composé de formule (1) tel que défini dans la revendication 1 ou d'une composition selon la revendication 2 à une plante, à une graine d'une plante, à l'emplacement de la plante ou de la graine ou au sol ou tout autre milieu de croissance de plantes.
